# EUROPEAN PATENT APPLICATION

(11) **EP 1 245 283 A2**
(43) Date of publication of application: **02.10.2002**
(21) Application number: 02007117.1
(22) Date of filing: 28.03.2002
(51) Int. Cl.: B01L 3/02

(54) **Adaptor for use with point-of-care testing cartridge**

(30) Priority: 30.03.2001 US 280404 P; 30.03.2001 US 280431 P
(71) Applicant: Becton Dickinson and Company, Franklin Lakes, New Jersey 07417-1880 (US)
(72) Inventor: Francavilla, Frank, Newton, New Jersey 07860 (US); Crawford, Jamieson William Maclean, New York, New York (US)
(74) Representative: Selting, Günther, Dipl.-Ing.

(57) **Abstract**

An adaptor is provided for use with a syringe and a blunt cannula to efficiently deliver a sample of blood to a point-of-care testing cartridge. The syringe includes a generally cylindrical tip with a passage that communicates with a fluid receiving chamber of the syringe. An annular or toroidal bead extends around the tip. The adaptor includes proximal and distal ends and a passage extending between the ends. The proximal end of the adaptor is configured to telescope with the tip of the syringe and to snap into engagement with the bead around the tip of the syringe. The distal end of the adaptor includes a Luer tip and an internally threaded Luer collar. A plastic cannula can be threadedly mounted to the distal end of the adaptor to facilitate delivery of a fluid specimen to an entry point of a point-of-care testing cartridge.

## Description

This application claims priority on U.S. Provisional Patent Appl. No. 60/280,404 and U.S. Provisional Patent Appl. No. 60/280,431 both of which were filed on March 30, 2001.

### BACKGROUND OF THE INVENTION

### 1. Field of the Invention

The subject invention relates to an adaptor to facilitate the transfer of a specimen from a syringe to a point-of-care testing cartridge.

### 2. Description of the Related Art

Many medical procedures require diagnostic tests to be performed on a sample of a patient's fluid. Fluid often is collected from a patient by employing a needle holder assembly and one or more evacuated tubes. Fluid also can be collected in a syringe. A syringe may be used with a metallic needle to obtain a fluid sample from a patient. However, syringes often are connected directly to an established arterial or venous line to obtain a fluid sample. The fluid collected in the syringe then may be transferred to a tube. The tubes are labeled carefully and shipped to a laboratory for analysis. The results of the laboratory analysis then are reported back to the health care provider. The results, of course, could be rushed in emergency situations, but absent an emergency would require more then one day between the time the sample is drawn from the patient to the time that the laboratory analysis is reported to the health care provider.

Devices have been developed for performing at least certain diagnostic tests on a sample of fluid at the point-of-care. The point-of-care diagnostic equipment includes a syringe for receiving a sample of fluid from a patient, a small disposable testing cartridge for receiving a portion of the fluid from the syringe and a portable clinical analyzer for analyzing the fluid and outputting the results. Combinations of testing cartridges and portable clinical analyzers are marketed in the United States by i-STAT Corporation, AVL Scientific Corporation and Diametrics Medical, Inc. The systems produced by these and other companies share certain common features. In particular, the testing cartridge of each system typically has a small rectangular housing about 1" x 2" and about .25" thick. The housing includes an internal reservoir with a volume of between about 40µl and 125µl. An inlet port extends through an external wall of the testing cartridge and communicates with the internal reservoir. The cartridge further includes contact pads and sensors that can be placed in communication with the portable clinical analyzer. An example of an i-STAT point-of-care testing cartridge is shown in U.S. Patent No. 5,638,828.

The prior art point-of-care testing systems are employed with a syringe that is used to draw a sample of fluid from a patient. The syringe then may be used to eject a portion of the fluid sample into the inlet port of the point-of-care testing cartridge. However, some testing cartridges are operative to automatically draw fluid from the syringe. The inlet port of the cartridge then is closed and the cartridge is placed in communication with the portable clinical analyzer for performing certain specified diagnostic tests on the sample of fluid in the cartridge. The analyzer then provides a very quick output of the test results without the need for sending the fluid sample to the laboratory.

Point-of-care testing systems provide several efficiencies over systems that require virtually all diagnostic tests to be performed at a location remote from the point-of-care. The small size of the testing cartridge facilitates storage and shipment of the cartridges while also contributing to the portability of the system. However, with regards to transferring a collected sample to the cartridge, the small cartridges can be very difficult to use. For example, alignment of the distal end of the syringe with the inlet port of the testing cartridge can be complicated and difficult. A misalignment or imprecise mating of the syringe with the inlet port of the testing cartridge can lead to a loss of a portion of the collected fluid sample. Additionally, it is difficult to use a syringe for accurately dispensing the proper volume of liquid. Too small a volume may prevent proper testing by the cartridge and the associated portable clinical analyzer. Too large a volume can cause splattering or spillage. Similarly an overfill can result in splatter when the cover of the point-of-care testing cartridge is closed. Fluid that is not delivered efficiently from the syringe into the inlet port of the testing cartridge create the potential for disease transmission. Similarly, a loss of fluid during the transfer from the syringe to the testing cartridge can leave an insufficient volume of fluid for performing the required diagnostic tests. An insufficient volume of fluid to perform the required tests can require the health care worker to return to the patient for a second sample of fluid. This is time consuming for the health care worker and traumatic for the patient. Additionally, some testing cartridges may require an insufficiently filled cartridge to be discarded and a new cartridge to be employed with the new sample of fluid. Thus, inefficiencies in the transfer of fluid from the syringe to the testing cartridge can generate excess costs for additional testing cartridges.

The direct transfer of fluid from a syringe to a testing cartridge can cause the syringe tip to close off the entry port and prevent venting of air from the testing cartridge. Thus bubbles are created. Bubbles reduce the volume of fluid and can affect test results.

IV access systems of tubes and fittings often are used for delivering liquid solutions to a patient. One such fitting is a blunt plastic tube with opposed proximal and distal ends and a lumen extending therebetween. Portions of the lumen adjacent the proximal end of the plastic fitting define a large tapered opening dimensioned to achieve a fluid-tight engagement with the tapered tip of a Luer fitting, such as the tip at the distal end of a syringe. The proximal end of the plastic fitting includes a pair of diametrically opposite lugs that are configured for engagement with the internal threads on a Luer collar. Threaded engagement of the lugs on the plastic fitting with the internal threads of the Luer collar cause the tip of the Luer fitting to telescope tightly into the tapered entry to the lumen of the plastic fitting. Thus, the prior art plastic fitting can achieve a secure mechanical connection with a Luer collar and a fluid-tight connection with the distal tip of the Luer fitting. The extreme distal tip of the plastic fitting terminates in a single axially aligned egress port with a diameter similar to the diameter of the lumen.

Plastic fittings have been used for a variety of medical purposes, including the injection of drugs into the fitting of an IV line. The plastic fittings, however, typically have not been used for phlebotomy or during any diagnostic procedures conducted after a sample of blood has been collected.

### SUMMARY OF THE INVENTION

The subject invention is directed to a Luer-lock adaptor for use with a point-of-care testing cartridge and a syringe assembly. The point-of-care testing cartridge may be a prior art testing cartridge as described above, or any yet-to-be developed testing cartridge for performing point-of-care diagnostic analysis on a collected specimen of blood or other bodily fluid. The testing cartridge comprises a housing having an internal reservoir for receiving a specimen to be tested. The housing may be substantially rectangular, with opposed top and bottom walls and a plurality of side walls. An entry port extends through the top wall and that communicates with the internal reservoir of the testing cartridge. The testing cartridge may further include contact pads and sensors that can be placed in communication with a portable clinical analyzer for performing point-of-care analysis of the collected specimen.

The syringe assembly that is used with the Luer-lock adaptor includes a body with opposed proximal and distal ends. A barrel extends distally from the proximal end of the body and defines a fluid receiving chamber that is widely open at the proximal end. A substantially cylindrical tip projects from the barrel to the distal end of the syringe body and includes a passage that communicates with the fluid receiving chamber. The outer surface of the tip includes an annular or toroidal bead intermediate the length of the tip.

The syringe assembly further includes a plunger that is slidably received in the open proximal end of the fluid receiving chamber defined by the syringe barrel. Distal movement of the plunger in the fluid receiving chamber will expel a fluid from the chamber and through the Luer tip. Proximal movement of the plunger in the chamber will draw fluid through the Luer tip and into the chamber.

The Luer-lock adaptor may be molded unitarily from plastic and includes a proximal end, a distal end and a lumen extending between the ends. The adaptor includes an inner tubular wall extending distally from the proximal end of the adaptor. The inner tubular wall surrounds and defines proximal portions of the lumen through the adaptor. The inner tubular wall has a substantially cylindrical or slightly tapered outer surface with a maximum diameter substantially equal to the inside diameter of the passage through the tip in the syringe. Thus, the inner tubular wall of the adaptor can be slid into the passage through the tip in the syringe.

The adaptor further includes an outer tubular wall that extends distally from the proximal end of the adaptor. The outer tubular wall is spaced concentrically outward from the inner tubular wall such that an annular space exists between the inner and outer tubular walls. The outer tubular wall includes an inner surfaced that defines an inside diameter substantially equal to the outside diameter of the cylindrical tip on the syringe. However, the inner surface of the outer tubular wall is characterized by an annular recess that is dimensioned and disposed to engage the annular bead on the tip of the syringe. With this arrangement, the tip of the syringe can be inserted into the annular space between the inner and outer tubular walls of the adaptor. Sufficient insertion of the tip into the annular space of the adaptor will cause the annular bead on the tip to snap securely into engagement with the annular recess on the inner surface of the outer tubular wall. Thus, the adaptor can be locked securely onto the annular tip of the syringe.

The distal end of the adaptor includes a Luer tip with a conically tapered outer surface that is dimensioned to mate with a conventional Luer fitting. The adaptor may further include a Luer collar that concentrically surrounds the Luer tip. The collar may include an array of internal threads for threaded engagement with lugs on a Luer fitting, such as the lugs at the proximal end of the above-described blunt plastic cannula. The adaptor can be snapped securely into engagement with the distal end of the syringe such that the lumen through the adaptor communicates with the passage through the cylindrical tip of the syringe.

In certain embodiments, a nose extends from the end of the Luer tip of the adaptor to the extreme distal end of the adaptor. The nose is substantially cylindrical and concentric with the lumen through the adaptor. Additionally, the nose is cross-sectionally substantially smaller than the distal end of the Luer tip and is sufficiently small for easy insertion into the entry port of a testing cartridge.

The Luer-lock adaptor may be used with a blunt plastic cannula that is molded unitarily from a plastic material and has opposite proximal and distal ends and a lumen extending between the ends. The proximal end of the blunt plastic cannula defines a female Luer fitting. The Luer fitting includes a conical entry to the lumen between the ends of the blunt plastic cannula. The conical entry is dimensioned and configured to mate with a conventional Luer tip. The proximal end of the blunt plastic cannula may further include diametrically opposite lugs that are configured for engagement with the internal threads of a Luer collar. The distal end of the blunt plastic cannula is longer and narrower than the tip of the syringe and is tapered sufficiently to pierce a septum across a fitting on an IV access system or fluid collection set. A rigid cap may be mounted over the distal end of the blunt plastic cannula.

The distal end of the adaptor can be placed in communication with appropriate structure for accessing a sample of a patient's blood. For example, the Luer tip and the Luer collar of the adaptor can be mated with appropriate fittings on an IV system for accessing a sample of a patient's blood or other bodily fluid. Alternatively, a conventional needle assembly can be mounted to the distal end of the adaptor so that a blood sample can be taken directly from an artery or vein of the patient or from a previously filled blood collection tube.

After an appropriate volume of fluid has been drawn into the fluid receiving chamber of the syringe, the syringe is separated from the source of the fluid. If a conventional needle assembly had been used, appropriate steps are taken for safely removing the needle assembly from the adaptor and depositing the used needle assembly in a sharps receptacle. The blunt plastic cannula then may be mounted to the distal end of the adaptor. This may involve threadedly engaging lugs on the blunt plastic cannula with the internal threads on the Luer collar of the adaptor. Alternatively, a blunt plastic cannula with no lugs can merely be urged axially over the Luer tip for fluid-tight frictional engagement with the Luer tip of the adaptor.

The cap on the blunt plastic cannula then is removed and the narrow slightly tapered distal end of the blunt plastic cannula is guided into the entry port of the testing cartridge. The plunger of the syringe assembly then is moved distally relative to the syringe barrel for urging an appropriate volume of the specimen from the fluid receiving chamber of the syringe into the testing cartridge. The syringe, the adaptor and the blunt plastic cannula then are separated from the testing cartridge and are deposited in an appropriate receptacle. The entry port of the testing cartridge then is covered, and the testing cartridge is presented to a portable digital analyzer for performance of the specified analytical tests. Alternatively, the testing cartridge may be mounted to the portable clinical analyzer before the fluid specimen is deposited in the testing cartridge. The above-described blunt plastic cannula need not be used if the adaptor has the small nose at the distal end. Thus, the nose can be directed into the entry port of the testing cartridge.

### DESCRIPTION OF THE DRAWINGS

FIG. 1 is an exploded perspective view of a syringe assembly and an adaptor in accordance with the subject invention.

FIG. 2 is a perspective view of the syringe assembly and the adaptor in their assembled condition.

FIG. 3 is a longitudinal cross-sectional view of the adaptor.

FIG. 4 is a perspective view of a point-of-care testing cartridge.

FIG. 5 is a side elevational view, partly in section, of a blunt plastic cannula and cap for use with the adaptor.

FIG. 6 is a cross-sectional view of the blunt plastic cannula.

FIG. 7 is a side elevational view of the blunt plastic cannula and cap mounted to the syringe and adaptor.

FIG. 8 is a side elevational view similar to FIG. 7, but showing the cap removed.

FIG. 9 is a perspective view showing the distal end of the blunt plastic cannula mounted to the entry port of the testing cartridge.

FIG. 10 is a longitudinal cross-sectional view of an alternate adaptor in accordance with the invention.

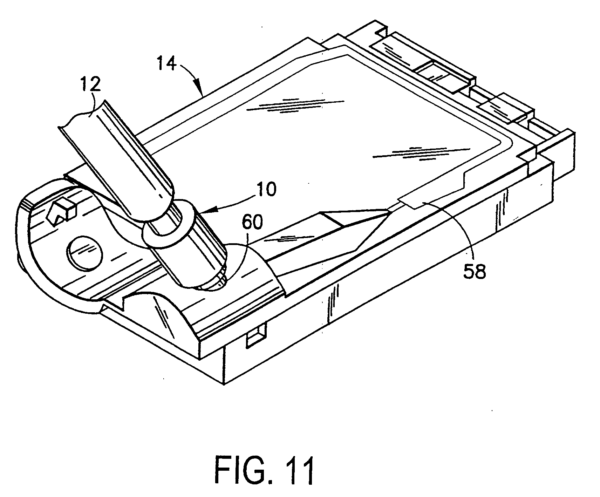

FIG. 11 is a perspective view showing the syringe and the adaptor of FIG. 10 mounted to the entry port of the testing cartridge.

### DETAILED DESCRIPTION

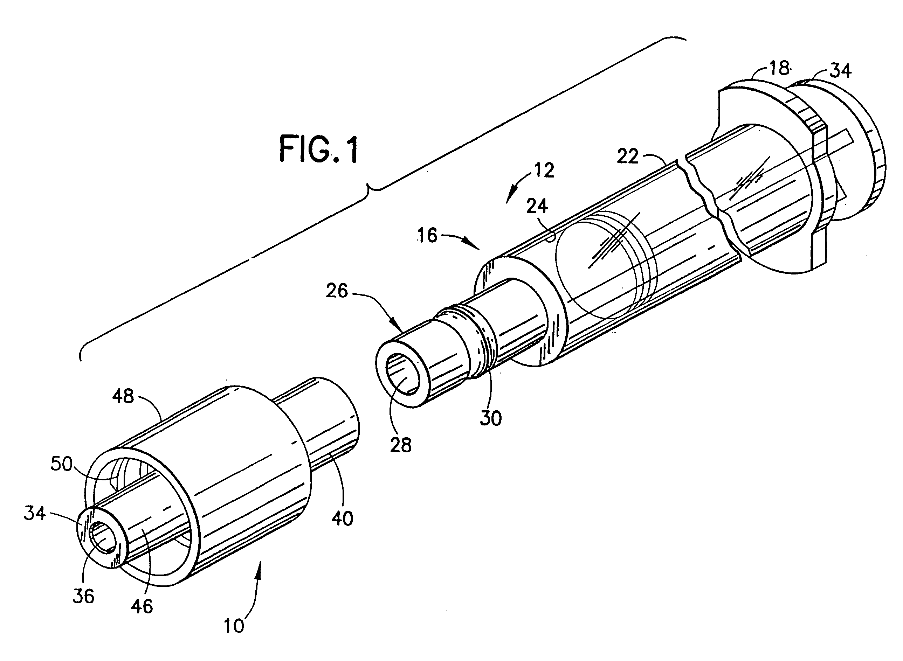

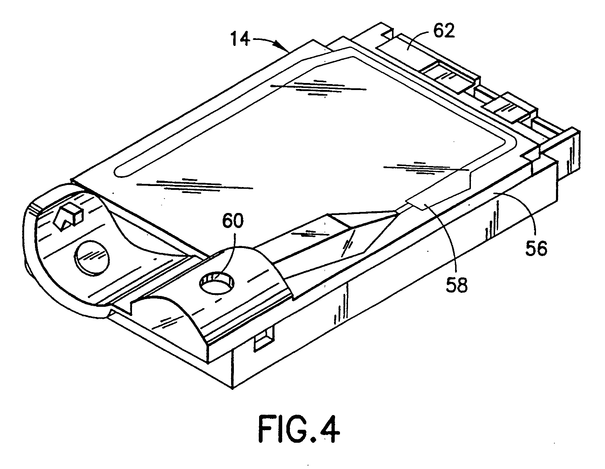

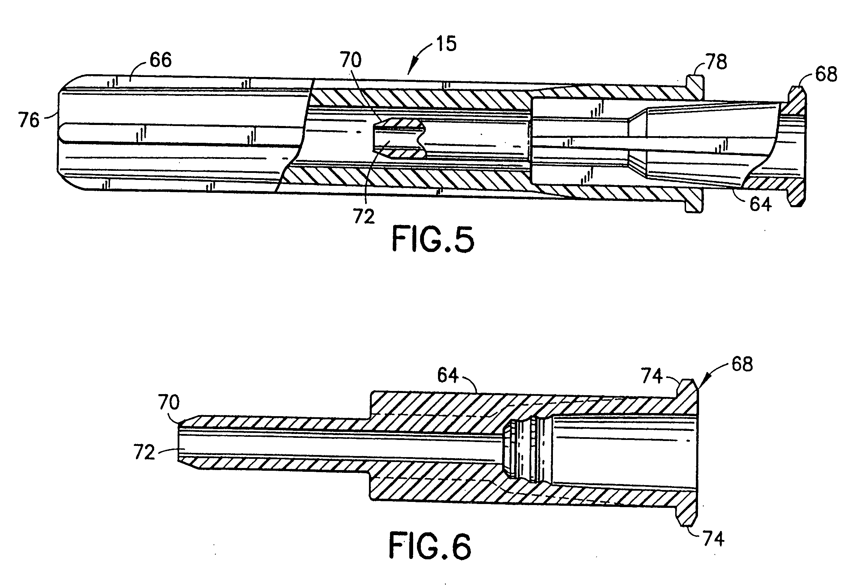

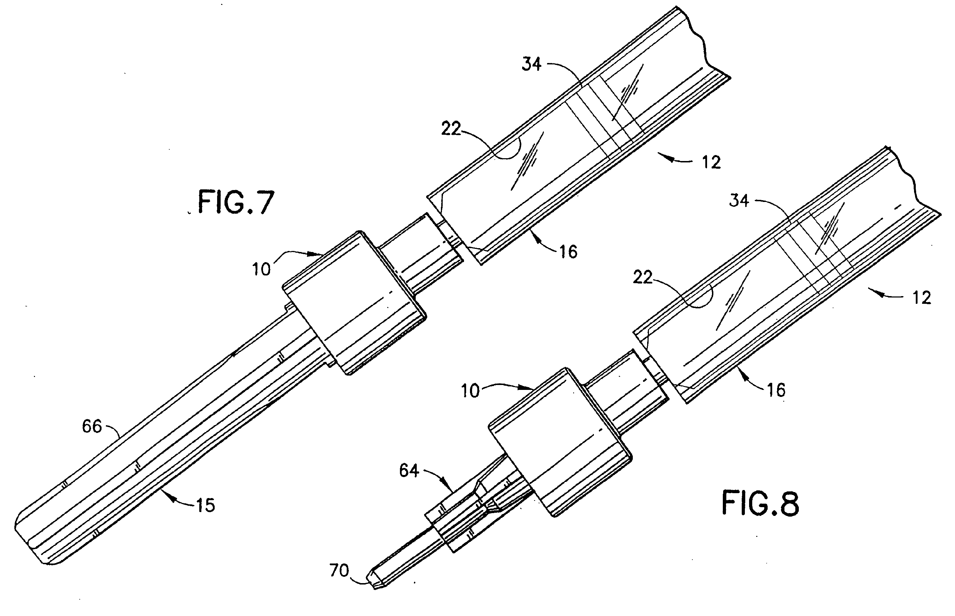

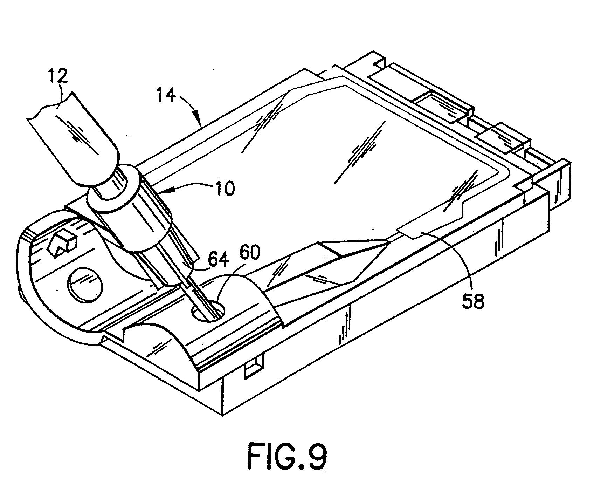

A Luer-lock adaptor in accordance with the subject invention is identified generally by the numeral **10** in FIGS. 1-3 and 7-9. Adaptor **10** is used with a syringe assembly **12,** as shown in FIGS. 1, 2 and 9, with a point-of-care testing cartridge **14,** as shown most clearly in FIG. 4 and with a blunt cannula assembly **15,** as shown most clearly in FIGS. 5 and 6.

Syringe assembly **12,** as shown in FIG. 1, includes a syringe body **16** having a proximal end **18** and a distal end **20.** A barrel **22** extends distally from proximal end **18** and defines a cylindrical fluid receiving chamber **24** that is widely open at proximal end **18.** A cylindrical tip **26** extends from barrel **22** to distal end **20** of syringe body **16.** Tip **26** is provided with a narrow cylindrical passage **28** that communicates with fluid receiving chamber **24** of barrel **22.** The outer cylindrical surface of cylindrical tip **26** is formed with a generally toroidal bead **30** extending around tip **26** at a location intermediate the length of tip **26.** Syringe assembly **12** further includes a plunger **34** slidably disposed in fluid receiving chamber **24** and in fluid-tight engagement with the cylindrical walls of chamber **24.** Plunger **34** can be moved alternately in proximal or distal directions for urging fluid through passage **28** in tip **26** and into or out of fluid receiving chamber **24.**

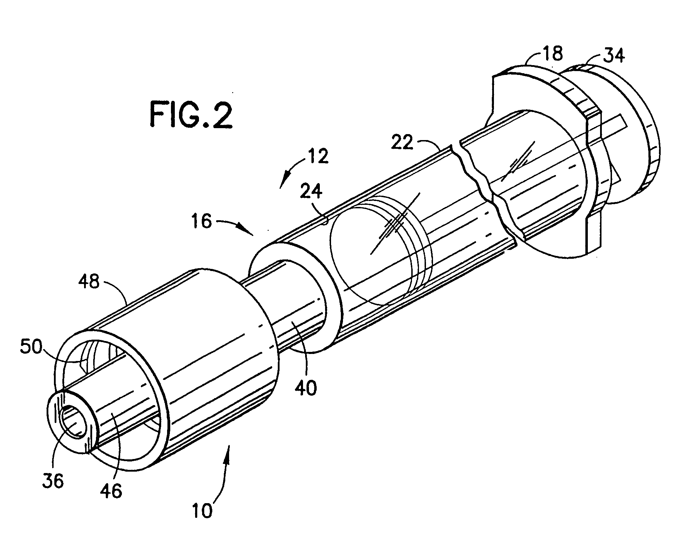

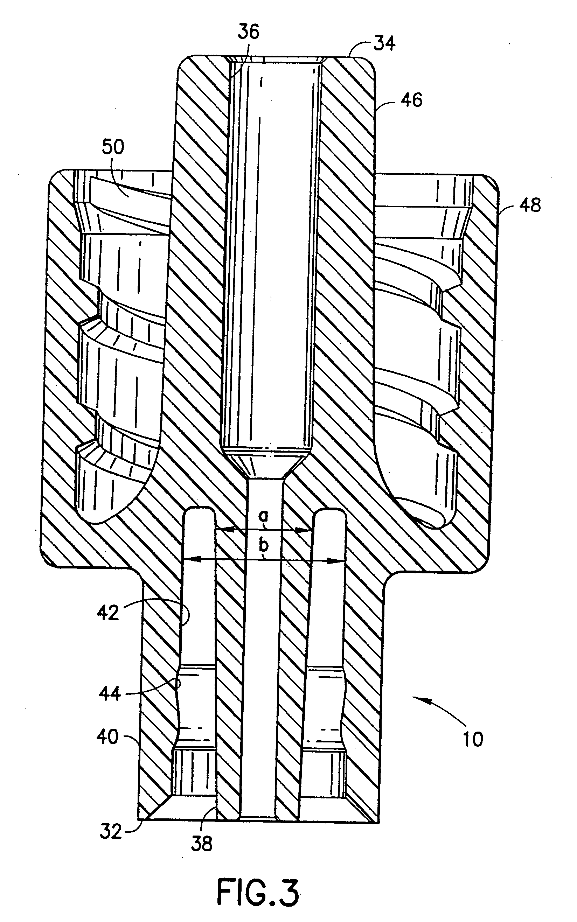

Syringe assembly **12** is used with adaptor **10,** as shown in FIGS. 1 and 2. Adaptor **10,** as shown most clearly in FIG. 3, is unitarily molded from a thermoplastic material and includes a proximal end **32,** a distal end **34** and a passage **36** extending between ends **32** and **34.** Adaptor **10** includes a tubular inner wall **38** that extends distally from proximal end **32.** Tubular inner wall **38** surrounds and defines proximal portions of passage **36.** The outer surface of tubular inner wall **38** is tapered slightly to facilitate complete mounting in passage **28** of tip **26.** Tubular inner wall **38** defines a maximum outside diameter **a** substantially equal to the inside diameter of passage **28** through tip **26** of syringe barrel **16.** Adaptor **10** further includes a tubular outer wall **40** that extends proximally from distal end **32.** Tubular outer wall **40** includes an inner surface **42** that is substantially concentric with tubular inner wall **38** and spaced outwardly from tubular inner wall **38.** Portions of inner surface **42** spaced from proximal end **32** are substantially cylindrical and define an inside diameter **b** substantially equal to the outside diameter of tip **26** at locations spaced from bead **30.** Inner surface **42** of tubular outer wall **40** is characterized by an annular recess **44** that is dimensioned and disposed to engage annular bead **30** of tip **26.** Thus, proximal end **32** of adaptor **10** can be mounted over tip **26** of syringe body **16** by urging tubular inner wall **38** into passage **28** and by urging tubular outer wall **40** over tip **26.** Sufficient movement of proximal end **32** of adaptor **10** onto tip **26** will cause bead **30** of tip **26** to snap into engagement with annular recess **44** of tubular outer wall **40.**

Adaptor **10** further includes a Luer tip **46** that extends proximally from distal end **34.** Luer tip **46** is dimensioned and configured for mating with a conventional Luer fitting, such as the Luer fitting existing on blunt cannula assembly **15,** as explained below. Adaptor **10** also includes a Luer collar **48** that concentrically surrounds Luer tip **42.** Luer collar **48** is characterized by an array of internal threads **50.**

Point-of-care testing cartridge **14** is shown in FIG. 4 and may be of any of several prior art designs, including those manufactured by i-STAT Corporation, Diametrics Medical, Inc., AVL Scientific Corporation or any other such testing cartridges that are available or become available. One such testing cartridge is disclosed in U.S. Patent No. 5,638,828, the disclosure of which is incorporated herein by reference.

Testing cartridge **14** includes a generally rectangular body **56** with a length of approximately 1.5-2.0 inches, a width of about 1.0 inches and a thickness of about 0.25 inches. A fluid reservoir **58** is formed inside body **56** of cartridge **14** and has a volume in the range of 40µl and 125µl. Body **56** further includes an entry port **60** that communicates with reservoir **58.** Entry port **60** is slightly tapered from a relatively large diameter portion externally on housing **56** to a relatively smaller cross-section closer to reservoir **58.** Testing cartridge **14** further includes contact pads and sensors **62** that can be placed in communication with a portable clinical analyzer for performing various point-of-care diagnostic tests on the sample of blood in the reservoir **58** and for providing various readout data that can be used by a health care technician at the point-of-care and/or at a remote location.

Blunt plastic cannula assembly **15** of FIG. 5 includes a blunt plastic cannula **64** and a plastic cap **66.** As shown more clearly in FIG. 6, the blunt plastic cannula **64** is unitarily molded from a plastic material and includes a proximal end **68** and a distal end **70** to define a length of slightly over 1.0 inches. A lumen **72** extends between ends **68** and **70.** Portions of lumen **72** adjacent proximal end **68** define a tapered entry can provide a fluid-tight frictional engagement with tapered Luer tip **46** of adaptor **10.** Proximal end **68** of blunt plastic cannula **64** is characterized further by diametrically opposite lugs **74** that are dimensioned and configured for engagement with internal threads **50** of Luer collar **48** of adaptor **10.** Thus, lugs **74** can be engaged threadedly with Luer collar **48** for urging the tapered proximal open end of lumen **72** into fluid-tight frictional engagement with tapered Luer tip **46** of adaptor **10.**

Portions of blunt plastic cannula **64** adjacent distal end **70** define a frustoconical taper having a maximum outside diameter significantly less than the inside diameter of entry port **60** of testing cartridge **14.** Blunt plastic cannula **64** continues at a substantially constant outside diameter to a location spaced from distal end **70**. Both the minimum outside diameter at distal end **70** and the maximum outside diameter at locations adjacent the frustoconical taper are substantially less than corresponding dimensions of Luer tip **46** of adaptor **10.** Furthermore, the degree of taper at distal end **70** of blunt plastic cannula **64** is greater than the taper existing on Luer tip **46** of adaptor **10.**

Returning to FIG. 5, cap **66** of plastic cannula assembly **15** includes a closed distal end **76** and an open proximal end **78.** Proximal end **78** of cap **66** can be telescoped over distal end **70** of fitting **64** and can be engaged frictionally with portions of blunt plastic cannula **64** between proximal and distal ends **68** and **70.**

Adaptor **10** is used by initially mounting proximal end **32** onto cylindrical tip **26** of syringe body **16** substantially as described with respect to the first embodiment. Adaptor **10** and syringe assembly **12** then can be used in a substantially conventional manner to access a sample of fluid from a patient. In this regard, adaptor **10** and syringe assembly **12** function substantially as a prior art syringe with a Luer tip and Luer collar unitarily molded as part of the syringe body.

After a sufficient volume of fluid has been obtained from the patient, adaptor **10** is separated from any needle assembly or fitting that had been employed to obtain the fluid sample. Blunt plastic cannula assembly **15** then is mounted to adaptor **10.** More particularly, lugs **74** at proximal end **68** of blunt plastic cannula **64** are threaded into engagement with internal threads **50** of Luer collar **48** on adaptor **10.** As a result, Luer tip **46** of adaptor **10** advances into secure fluid-tight engagement with entry to passage **72** at proximal end **68** of blunt plastic cannula **64.** Plastic cap **66** then is separated from blunt cannula **64** to expose distal end **70** of blunt cannula **64.** Distal end **70** of blunt cannula **64** then is guided into entry port **60** of testing cartridge **12.** Plunger **32** of syringe assembly **12** then is moved distally a sufficient distance to direct an appropriate volume of fluid through entry port **60** and into reservoir **58** of testing cartridge **12.** The assembly consisting of blunt cannula **64,** adaptor **10** and syringe **12** then are separated from testing cartridge **12** and discarded in an appropriate safe manner. The cover of testing cartridge **12** then is rotated over entry port **60,** and sensor pads **62** are engaged with the portable clinical analyzer (not shown) for performing a selected analysis on the sample of fluid deposited in testing cartridge **12.**

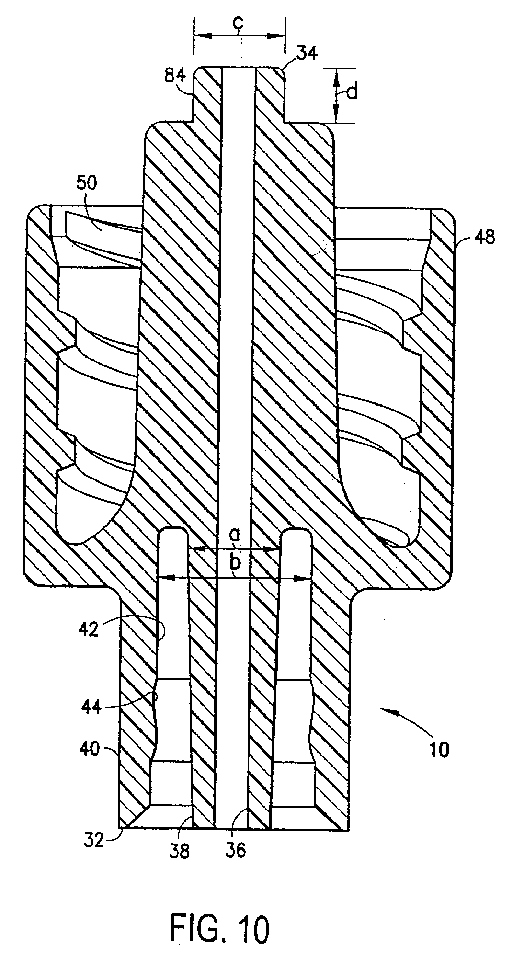

An alternate adaptor in accordance with the invention is illustrated in FIG. 10. The alternate adaptor is substantially identical to adaptor **10** of FIGS. 1-3, and accordingly components of the alternate adaptor that are identical to components in the adaptor of FIGS. 1-3 are identified by the same reference numerals, and a detailed description of those identical components is not provided. Alternate adaptor **10** differs from the first embodiment in that a nose **84** is defined at distal end **34** of adaptor **10** and effectively defines a profiled extension of Luer tip **46.** Nose **84** is substantially cylindrical and defines an outside diameter **c** which is approximately one half the outside diameter at the distal end of Luer tip **46.** Outside diameter **c** of nose **84** is approximately 0.05-0.15 inches and is sufficiently small to facilitate insertion into entry port of testing cartridge **14,** as explained further below. Nose **84** defines a length **d** of approximately 0.1 inches. Length **d** is sufficient to facilitate visual guiding of nose **84** toward the entry port of testing cartridge **14,** as explained herein. However, length **d** is selected to be sufficiently short for preventing passage **36** from being blocked by contact between nose **64** and internal structure on testing cartridge **14.**

The adaptor **10** is used by initially mounting proximal end **32** onto cylindrical tip **26** of syringe body **16** substantially as described with respect to the first embodiment. Adaptor **10** and syringe assembly **12** then can be used in a substantially conventional manner to access a sample of fluid from a patient. In this regard, adaptor **10** and syringe assembly **12** function substantially as a prior art syringe with a Luer tip and Luer collar unitarily molded as part of the syringe body. Nose **84** does not impede connection of Luer tip **46** with Luer fitting.

After a sufficient volume of fluid has been obtained from the patient, adaptor **10** and syringe assembly **12** are separated from any needle assembly or fitting that had been employed to obtain the blood sample. Nose **84** of adaptor **10** then is guided into entry port **60** of testing cartridge **14.** As explained above, diameter, **c** of nose **84** is sufficiently smaller than entry port **60** to facilitate slidable insertion. Additionally, length **d** of nose **64** is sufficiently long to facilitate visual alignment of nose **84** with entry port **60,** but is sufficiently short to prevent nose **64** from contacting internal portions of testing cartridge **14** in a manner that would block passage **36** through adaptor **10.** Plunger **32** of syringe assembly **12** then is moved distally a sufficient distance to direct an appropriate volume of fluid through entry port **60** and into reservoir **58** of testing cartridge **14.** The assembly consisting of an adaptor **10** and syringe **12** then are separated from testing cartridge **14** and discarded in an appropriate safe manner. The cover of testing cartridge **14** then is rotated over entry port **60,** and sensor pads **62** are engaged with the portable clinical analyzer for performing a selected analysis on the sample of fluid deposited in testing cartridge **14.**

## Claims

1. An adaptor unitarily molded from a plastic material and having a proximal end, a distal end and a passage extending between said ends, a frustoconical tip substantially adjacent said distal end and defining portions of said passage adjacent said distal end, a collar concentrically surrounding portions of said tip and spaced outwardly from said tip, said collar including an array of internal threads, said proximal end including an inner tubular wall surrounding and defining portions of said passage adjacent said proximal end, an outer tubular wall surrounding said inner tubular wall and spaced therefrom such that a substantially annular opening extends into said proximal end between said inner and outer tubular walls.

2. The adaptor of Claim 1, wherein said inner tubular wall is substantially cylindrical.

3. The adaptor of Claim 1, wherein said outer tubular wall includes an inner surface, said surface being **characterized by** an annular recess.

4. The adaptor of Claim 1, further comprising a cylindrical nose at said distal end and surrounding portions of said passage at said distal end, said nose defining a selected outside diameter, said frustoconical tip having an outside diameter adjacent said nose that is larger than said outside diameter of said nose.

5. The adaptor of Claim 4, wherein said outside diameter of said nose is approximately 0.05-0.1 inches.

6. The adaptor of Claim 5, wherein said nose defines an axial length approximately equal to said outside diameter.

7. A syringe and adaptor assembly comprising:
a syringe body having a proximal end, a distal end and a fluid receiving chamber therebetween, said fluid receiving chamber being open at said proximal end of said syringe body, a tip defined at said distal end of said syringe body, a tip passage extending through said tip and into communication with said fluid receiving chamber, said tip having an outer circumferential surface formed with an annular bead thereon; and
an adaptor having opposed proximal and distal ends and an adaptor passage extending between said ends, said distal end of said adaptor defining a Luer fitting, said proximal end of said adaptor comprising an inner tubular wall surrounding and defining a portion of said adaptor passage, said inner tubular wall being dimensioned for sliding insertion into said tip passage of said syringe body, said proximal end of said adaptor further comprising an outer tubular wall surrounding and spaced from said inner tubular wall, said outer tubular wall defining an inside diameter substantially conforming to external dimensions of said tip of said syringe body, and including an annular recess dimensioned and disposed for snapped engagement with said annular bead on said tip of said syringe body.

8. The assembly of Claim 7, wherein said Luer fitting of said adaptor comprises a conically tapered Luer tip concentrically surrounding portions of said adaptor passage at said distal end of said adaptor and an internally threaded Luer collar surrounding and spaced from said Luer tip.

9. A syringe assembly for delivering a fluid sample to a point-of-care testing cartridge, said testing cartridge having an internal reservoir and an entry port communicating with said internal reservoir, said assembly comprising:
a syringe having a proximal end and a distal end, a fluid receiving chamber defined between said ends of said syringe, said chamber being open at said proximal end of said syringe, a substantially cylindrical tip projecting at said distal end and having a tip passage extending from said fluid receiving chamber to said distal end of said syringe;
an adaptor having a proximal end, a distal end and an adaptor passage extending between said ends, said distal end of said adaptor comprising a Luer tip surrounding said passage, said proximal end of said adaptor having an inner tubular wall dimensioned for slidable insertion in said tip passage of said syringe, an outer tubular wall dimensioned for surrounding and engaging outer circumferential portions of said tip of said syringe; and
a blunt cannula having proximal and distal ends and a lumen extending therebetween, said proximal end of said blunt cannula defining a Luer fitting configured for fluid-tight engagement with said Luer tip at said distal end of said adaptor, said distal end of said blunt cannula being cross-sectionally dimensioned for insertion into said entry portion of said testing cartridge.

10. An assembly comprising:
a point-of-care testing cartridge having an entry port, said entry port defining a selected internal diameter; and
an adaptor unitarily molded from a plastic material and having opposed proximal and distal ends and a passage extending between said ends, said proximal end of said adaptor comprising an inner tubular wall surrounding and defining portion of said passage adjacent said proximal end and an outer tubular wall surrounding and spaced from said inner tubular wall such that an annular space extends into said proximal end between said inner and outer tubular walls, a Luer tip disposed distally of said inner and outer tubular walls, a Luer collar surrounding said Luer tip, said Luer collar having an array of internal threads, and a nose projecting distally from said Luer tip to said distal end of said adaptor, said nose defining an outside cross-sectional dimension less than said inside diameter of said entry port of said testing cartridge, whereby said nose facilitates alignment and insertion of said adaptor into said entry port.
